# EUROPEAN PATENT APPLICATION

(11) **EP 4 772 184 A1**
(43) Date of publication of application: **08.07.2026**
(21) Application number: 23951060.5
(22) Date of filing: 04.09.2023
(51) Int. Cl.: A61K 35/20, A61P 1/00, A61P 1/04

(54) **MEV AND USE THEREOF IN PREPARATION OF DRUG FOR PREVENTING AND/OR TREATING GASTRIC ULCER**

(71) Applicant: Panexo Biosciences AB, 411 26 Göteborg (SE)
(72) Inventor: WANG, Yi, Beijing 100141 (CN); DONG, Yanan, Beijing 100141 (CN); LI, Jun, Beijing 100141 (CN); LI, Yanfang, Beijing 100141 (CN); ZHAO, Taiyun, Beijing 100141 (CN)
(74) Representative: Casalonga
(86) International application number: PCT/CN2023/116644
(87) International publication number: WO 2025/050229

(57) **Abstract**

Provided are a milk-derived extracellular vesicle (mEV) and a use thereof in preparation of a drug for preventing and/or treating gastric ulcer. The mEV comprises a protein component beneficial to gastric mucosa reconstruction and immunoregulation and an miRNA closely related to immunoregulation and anti-infection functions.

## Description

### FIELD

The present invention relates to the field of biomedicine, in particular to milk-derived extracellular vesicles (milk EV, mEV) and use thereof in the manufacture of a medicament for preventing and/or treating gastric ulcer.

### BACKGROUND

Clinical treatments for gastric ulcer mainly include three strategies: suppression of gastric acid, suppression of *Helicobacter pylori,* and protection of the gastric mucosa. Gastric acid-suppressing drugs include: 1) antacids (such as aluminum hydroxide, aluminium magnesium carbonate, calcium carbonate, and magnesium oxide); 2) H2 receptor antagonists (such as famotidine, roxatidine, lafutidine and nizatidine); 3) proton pump inhibitors (lansoprazole, rabeprazole, omeprazole, pantoprazole and esomeprazole). Drugs for suppressing *Helicobacter pylori* include clarithromycin, metronidazole and amoxicillin. Gastric mucosal protective agents include bismuth potassium citrate, sucralfate, colloidal bismuth tartrate capsules and teprenone.

Due to modern high-pressure, fast-paced lifestyle and unhealthy dietary habits, gastric ulcer becomes prevalent, for treating which long-term administration of small-molecule chemical drugs would result in cumulative adverse effects and substantial safety risks.

Researchers have begun to explore biotechnological approaches (such as cell therapy) for the prevention or treatment of the gastric ulcer. Cells and cell derivatives have good safety and significant efficacy. For example, extracellular vesicles extracted from cultured endothelial progenitor cells can be used via injection administration for the treatment of hemorrhagic diseases (including gastric ulcer).

Currently, issues with existing cell therapies include:
(1) The source of endothelial progenitor cells is limited by ethical issues and cost, making scale-up culture difficult;
(2) Due to the limited culture scale of endothelial progenitor cells, the production scale of exosomes from endothelial progenitor cells is also limited, and its cost is high;
(3) The existing technologies require intravenous injection for administration, which may generally lead to poor compliance of patients with gastric ulcers in clinical practice; and
(4) The progenitor cells, as pluripotent stem cells, possess a certain degree of long-term proliferation ability, exosomes derived from which may carry bioactive components that can promote cell differentiation and proliferation, and thus would cause a risk of carcinogenicity after being injected into the body.

Therefore, for the prevention and treatment of the gastric ulcer, it is of great practical significance to develop a biotechnological product which is safe, effective, and convenient for clinical application.

### SUMMARY

In view of foregoing issues, the present invention provides a milk-derived extracellular vesicle (milk EV, mEV), extracted from fresh skimmed milk and cheese whey as raw materials, for the prevention and treatment of the gastric ulcer.

In order to achieve the objective, the present invention provides the following technical solutions:
In a first aspect, the present invention provides use of an mEV in the manufacture of a medicament for preventing and/or treating gastric ulcer.

In some specific embodiments of the present invention, the method of preparing the mEV comprises:
step 1: pre-treating a raw material, filtering the raw material through a 400-mesh filter cloth, then filtering through a 0.8/0.45 µm capsule filter, and collecting a filtrate,
step 2: concentrating the filtrate with a 300 kD tangential flow filtration membrane cassette, and diafiltering to exchange buffer into 1×PBS,
step 3: purifying the concentrated sample through Capto Core 700 (Cytiva) for refinement and removal of a free impurity protein, and
step 4: concentrating with a 750 kD hollow fiber tangential flow filtration column into a target volume or concentration.

In some specific embodiments of the present invention, the raw material includes fresh skimmed milk or cheese whey;
when the raw material is fresh skimmed milk, the pre-treating comprises preparing a 3 M ammonium sulfate solution, slowly adding an equal volume of the fresh skimmed milk to the ammonium sulfate solution with stirring to achieve a final concentration of ammonium sulfate of 1.5 M, and standing at room temperature for 3 h to allow precipitation;
when the raw material is cheese whey, the pre-treating comprises centrifuging the cheese whey at 5500 rpm for 10 min and removing a floating fat layer.

In some specific embodiments of the present invention, the mEV comprises one or at least two of glycoprotein, extracellular vesicle (EV) marker protein, apolipoprotein, immune-associated protein and cytokine.

In some specific embodiments of the present invention, the mEV comprises one or at least two of GLYCAM1, BTN1A1, LGB, MFGE8, CD36, XDH, ABCG2, FABP3, SLC34A2, MUC15, FASN, IDH1, CD9, ENPP3, MUC1, APOA1, ANXA5, RAB18, BPTI, PIGR, PPIA, PLIN2, GDI2, NT5E, ALB, EZR, HSPA8, RAC1, CYB5R3, RAB7A, RAB1B, TLR2, CDC42, CD63, CD46, ARHGDIA, EEF1A1, APOE, RHOA, GPD1, EEF2 or PTPA.

In some specific embodiments of the present invention, the mEV comprises:

| Proteins | Minimum Contents | Maximum Contents |
|---|---|---|
| GLYCAM1 | 5.3% | 36.2% |
| BTN1A1 | 9.4% | 19.3% |
| LGB | 1.8% | 8.4% |
| MFGE8 | 5.0% | 21.6% |
| CD36 | 3.3% | 7.9% |
| XDH | 3.7% | 8.8% |
| ABCG2 | 1.9% | 4.7% |
| FABP3 | 2.5% | 3.9% |
| SLC34A2 | 1.5% | 4.0% |
| MUC15 | 1.1% | 3.6% |
| FASN | 1.7% | 6.3% |
| IDH1 | 1.5% | 2.7% |
| CD9 | 0.6% | 2.4% |
| ENPP3 | 0.4% | 1.6% |
| MUC1 | 0.4% | 1.9% |
| APOA1 | 0.1% | 3.3% |
| ANXA5 | 0.3% | 1.0% |
| RAB18 | 0.3% | 0.9% |
| BPTI | 0.5% | 2.3% |
| PIGR | 0.4% | 1.1% |
| PPIA | 0.2% | 0.7% |
| PLIN2 | 0.6% | 3.1% |
| GDI2 | 0.1% | 0.5% |
| NT5E | 0.3% | 0.5% |
| ALB | 0.0% | 0.7% |
| EZR | 0.1% | 1.1% |
| HSPA8 | 0.2% | 0.6% |
| RAC1 | 0.1% | 0.4% |
| CYB5R3 | 0.1% | 0.4% |
| RAB7A | 0.1% | 0.4% |
| RAB1B | 0.1% | 0.3% |
| TLR2 | 0.1% | 0.4% |
| CDC42 | 0.1% | 0.3% |
| CD63 | 0.0% | 0.7% |
| CD46 | 0.1% | 0.3% |
| ARHGDIA | 0.1% | 1.5% |
| EEF1A1 | 0.1% | 0.5% |
| APOE | 0.1% | 1.2% |
| RHOA | 0.1% | 0.2% |
| GPD1 | 0.1% | 0.2% |
| EEF2 | 0.1% | 0.3% |
| PTPA | 0.1% | 0.2%; |

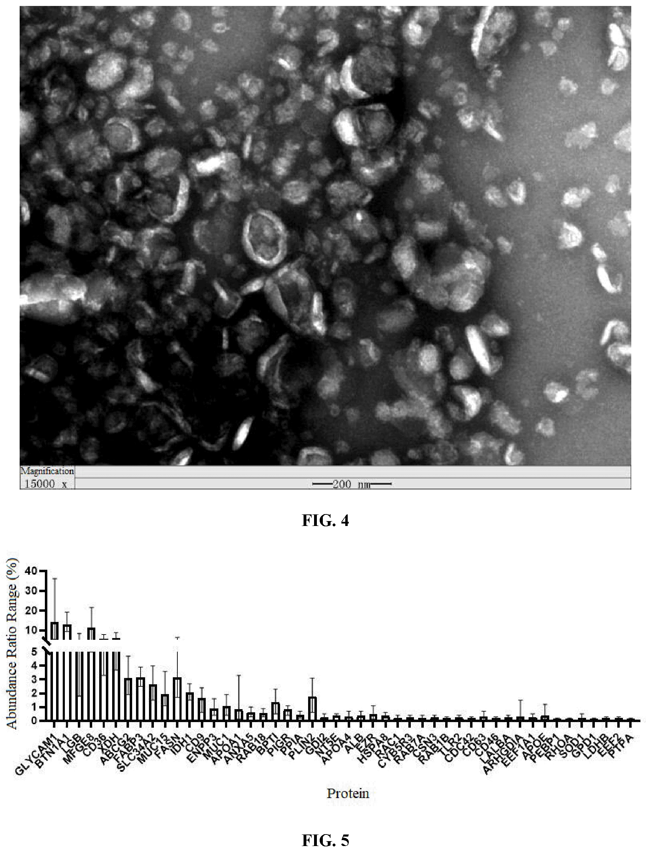

the proteins in the mEV and their abundance ratios are shown in FIG. 5.

In some specific embodiments of the present invention, the mEV comprises:

| | |
|---|---|
| GLYCAM1 | 10.6% |
| BTN1A1 | 9.4% |
| LGB | 7.2% |
| MFGE8 | 6.6% |
| CD36 | 6.3% |
| XDH | 6.0% |
| ABCG2 | 4.5% |
| FABP3 | 3.9% |
| SLC34A2 | 3.8% |
| MUC15 | 3.6% |
| FASN | 3.1% |
| IDH1 | 2.3% |
| CD9 | 2.2% |
| ENPP3 | 1.4% |
| MUC1 | 1.1% |
| APOA1 | 1.0% |
| ANXA5 | 0.9% |
| RAB18 | 0.9% |
| BPTI | 0.8% |
| PIGR | 0.7% |
| PPIA | 0.7% |
| PLIN2 | 0.6% |
| GDI2 | 0.5% |
| NT5E | 0.5% |
| APOA4 | 0.5% |
| ALB | 0.4% |
| EZR | 0.4% |
| HSPA8 | 0.4% |
| RAC1 | 0.4% |
| CYB5R3 | 0.4% |
| RAB7A | 0.4% |
| CSN3 | 0.4% |
| RAB1B | 0.3% |
| TLR2 | 0.3% |
| CDC42 | 0.3% |
| CD63 | 0.3% |
| CD46 | 0.3% |
| LALBA | 0.3% |
| ARHGDIA | 0.3% |
| EEF1A1 | 0.3% |
| APOE | 0.3% |
| PEBP1 | 0.2% |
| RHOA | 0.2% |
| SOD1 | 0.2% |
| GPD1 | 0.2% |
| LDHB | 0.2% |
| EEF2 | 0.2% |
| PTPA | 0.2%; |

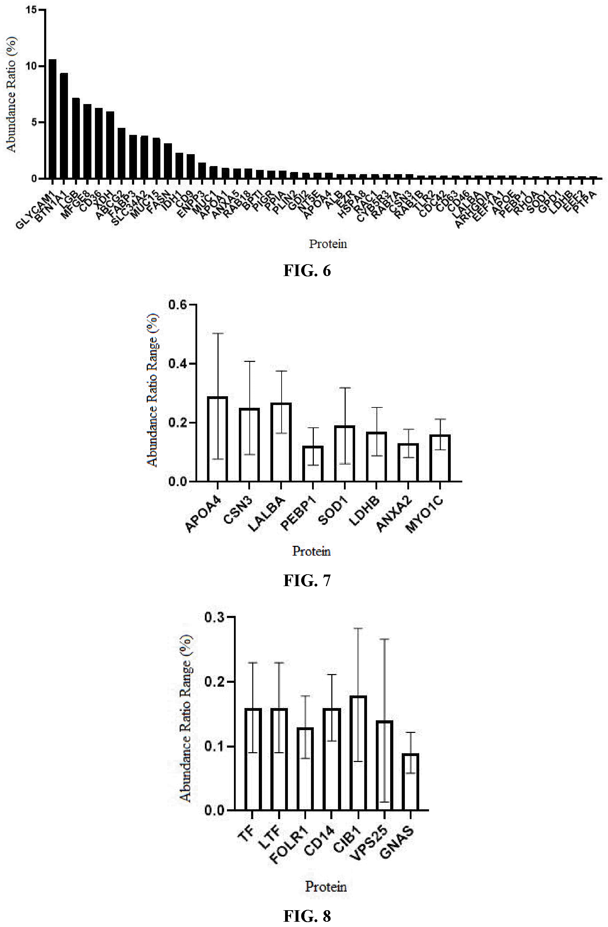

the proteins in the mEV and their abundance ratios are shown in in FIG. 6.

In some specific embodiments of the present invention, the mEV further comprises:
(I) one or at least two of APOA4, CSN3, LALBA, PEBP1, SOD1, LDHB, ANXA2 or MYO1C; and/or
(II) one or at least two of TF, LTF, FOLR1, CD14, CIB1, VPS25 or GNAS.

In some specific embodiments of the present invention, the mEV further comprises:

| Proteins | Minimum Contents | Maximum Contents |
|---|---|---|
| APOA4 | 0.0% | 0.7% |
| CSN3 | 0.0% | 0.4% |
| LALBA | 0.1% | 0.4% |
| PEBP1 | 0.0% | 0.2% |
| SOD1 | 0.1% | 0.5% |
| LDHB | 0.1% | 0.3% |
| ANXA2 | 0.1% | 0.2% |
| MYO1C | 0.1% | 0.2%; |

the proteins in the mEV and their abundance ratios are shown in FIG. 7;
alternatively, the mEV further comprises:

| Proteins | Minimum Contents | Maximum Contents |
|---|---|---|
| TF | 0.1% | 0.3% |
| LTF | 0.0% | 0.2% |
| FOLR1 | 0.1% | 0.2% |
| CD14 | 0.1% | 0.2% |
| CIB1 | 0.1% | 0.4% |
| VPS25 | 0.0% | 0.4% |
| GNAS | 0.0% | 0.1%; |

the proteins in the mEV and their abundance ratios are shown in FIG. 8.

In some specific embodiments of the present invention, the mEV further comprises:

| | |
|---|---|
| APOA4 | 0.5% |
| CSN3 | 0.1% |
| LALBA | 0.3% |
| PEBP1 | 0.2% |
| SOD1 | 0.2% |
| LDHB | 0.2% |
| ANXA2 | 0.2% |
| MYO1C | 0.2%; |

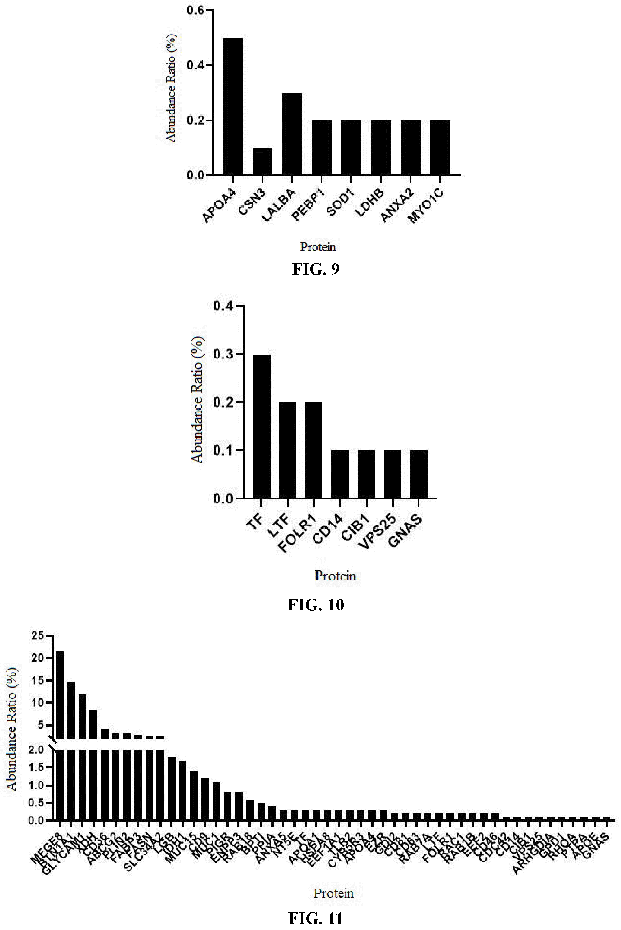

the proteins in the mEV and their abundance ratios are shown in FIG. 9;
alternatively, the mEV further comprises:

| | |
|---|---|
| TF | 0.3% |
| LTF | 0.2% |
| FOLR1 | 0.2% |
| CD14 | 0.1% |
| CIB1 | 0.1% |
| VPS25 | 0.1% |
| GNAS | 0.1%; |

the proteins in the mEV and their abundance ratios are shown in FIG. 10.

In a second aspect, the present invention further provides an mEV, which is manufactured by a method comprising:
step 1: pre-treating a raw material, filtering the raw material through a 400-mesh filter cloth, then filtering through a 0.8/0.45 µm capsule filter, and collecting a filtrate,
step 2: concentrating the filtrate with a 300 kD tangential flow filtration membrane cassette, and diafiltering to exchange buffer into 1×PBS,
step 3: purifying the concentrated sample through Capto Core 700 (Cytiva) for refinement and removal of a free impurity protein, and
step 4: concentrating with a 750 kD hollow fiber tangential flow filtration column into a target volume or concentration.

In some specific embodiments of the present invention, the raw material includes fresh skimmed milk or cheese whey;
when the raw material is fresh skimmed milk, the pre-treating comprises preparing a 3 M ammonium sulfate solution, slowly adding an equal volume of the fresh skimmed milk to the ammonium sulfate solution with stirring to achieve a final concentration of ammonium sulfate of 1.5 M, and standing at room temperature for 3 h to allow precipitation;
when the raw material is cheese whey, the pre-treating comprises centrifuging the cheese whey at 5500 rpm for 10 min, and removing a floating fat layer.

In some specific embodiments of the present invention, the mEV comprises one or at least two of glycoprotein, EV marker protein, apolipoprotein, immune-associated protein and cytokine.

In some specific embodiments of the present invention, the mEV comprises one or at least two of GLYCAM1, BTN1A1, LGB, MFGE8, CD36, XDH, ABCG2, FABP3, SLC34A2, MUC15, FASN, IDH1, CD9, ENPP3, MUC1, APOA1, ANXA5, RAB18, BPTI, PIGR, PPIA, PLIN2, GDI2, NT5E, ALB, EZR, HSPA8, RAC1, CYB5R3, RAB7A, RAB1B, TLR2, CDC42, CD63, CD46, ARHGDIA, EEF1A1, APOE, RHOA, GPD1, EEF2 or PTPA.

In some specific embodiments of the present invention, the mEV comprises:

| Proteins | Minimum Contents | Maximum Contents |
|---|---|---|
| GLYCAM1 | 5.3% | 36.2% |
| BTN1A1 | 9.4% | 19.3% |
| LGB | 1.8% | 8.4% |
| MFGE8 | 5.0% | 21.6% |
| CD36 | 3.3% | 7.9% |
| XDH | 3.7% | 8.8% |
| ABCG2 | 1.9% | 4.7% |
| FABP3 | 2.5% | 3.9% |
| SLC34A2 | 1.5% | 4.0% |
| MUC15 | 1.1% | 3.6% |
| FASN | 1.7% | 6.3% |
| IDH1 | 1.5% | 2.7% |
| CD9 | 0.6% | 2.4% |
| ENPP3 | 0.4% | 1.6% |
| MUC1 | 0.4% | 1.9% |
| APOA1 | 0.1% | 3.3% |
| ANXA5 | 0.3% | 1.0% |
| RAB18 | 0.3% | 0.9% |
| BPTI | 0.5% | 2.3% |
| PIGR | 0.4% | 1.1% |
| PPIA | 0.2% | 0.7% |
| PLIN2 | 0.6% | 3.1% |
| GDI2 | 0.1% | 0.5% |
| NT5E | 0.3% | 0.5% |
| ALB | 0.0% | 0.7% |
| EZR | 0.1% | 1.1% |
| HSPA8 | 0.2% | 0.6% |
| RAC1 | 0.1% | 0.4% |
| CYB5R3 | 0.1% | 0.4% |
| RAB7A | 0.1% | 0.4% |
| RAB1B | 0.1% | 0.3% |
| TLR2 | 0.1% | 0.4% |
| CDC42 | 0.1% | 0.3% |
| CD63 | 0.0% | 0.7% |
| CD46 | 0.1% | 0.3% |
| ARHGDIA | 0.1% | 1.5% |
| EEF1A1 | 0.1% | 0.5% |
| APOE | 0.1% | 1.2% |
| RHOA | 0.1% | 0.2% |
| GPD1 | 0.1% | 0.2% |
| EEF2 | 0.1% | 0.3% |
| PTPA | 0.1% | 0.2%; |

the proteins in the mEV and their abundance ratios are shown in FIG. 5.

In some specific embodiments of the present invention, the mEV comprises:

| | |
|---|---|
| GLYCAM1 | 10.6% |
| BTN1A1 | 9.4% |
| LGB | 7.2% |
| MFGE8 | 6.6% |
| CD36 | 6.3% |
| XDH | 6.0% |
| ABCG2 | 4.5% |
| FABP3 | 3.9% |
| SLC34A2 | 3.8% |
| MUC15 | 3.6% |
| FASN | 3.1% |
| IDH1 | 2.3% |
| CD9 | 2.2% |
| ENPP3 | 1.4% |
| MUC1 | 1.1% |
| APOA1 | 1.0% |
| ANXA5 | 0.9% |
| RAB18 | 0.9% |
| BPTI | 0.8% |
| PIGR | 0.7% |
| PPIA | 0.7% |
| PLIN2 | 0.6% |
| GDI2 | 0.5% |
| NT5E | 0.5% |
| APOA4 | 0.5% |
| ALB | 0.4% |
| EZR | 0.4% |
| HSPA8 | 0.4% |
| RAC1 | 0.4% |
| CYB5R3 | 0.4% |
| RAB7A | 0.4% |
| CSN3 | 0.4% |
| RAB1B | 0.3% |
| TLR2 | 0.3% |
| CDC42 | 0.3% |
| CD63 | 0.3% |
| CD46 | 0.3% |
| LALBA | 0.3% |
| ARHGDIA | 0.3% |
| EEF1A1 | 0.3% |
| APOE | 0.3% |
| PEBP1 | 0.2% |
| RHOA | 0.2% |
| SOD1 | 0.2% |
| GPD1 | 0.2% |
| LDHB | 0.2% |
| EEF2 | 0.2% |
| PTPA | 0.2%; |

the proteins in the mEV and their abundance ratios are shown in FIG. 6.

In some specific embodiments of the present invention, the mEV further comprises:
(I) one or at least two of APOA4, CSN3, LALBA, PEBP1, SOD1, LDHB, ANXA2 or MYO1C; and/or
(II) one or at least two of TF, LTF, FOLR1, CD14, CIB1, VPS25 or GNAS.

In some specific embodiments of the present invention, the mEV further comprises:

| Proteins | Minimum Contents | Maximum Contents |
|---|---|---|
| APOA4 | 0.0% | 0.7% |
| CSN3 | 0.0% | 0.4% |
| LALBA | 0.1% | 0.4% |
| PEBP1 | 0.0% | 0.2% |
| SOD1 | 0.1% | 0.5% |
| LDHB | 0.1% | 0.3% |
| ANXA2 | 0.1% | 0.2% |
| MYO1C | 0.1% | 0.2%; |

the proteins in the mEV and their abundance ratios are shown in FIG. 7;
alternatively, the mEV further comprises:

| Proteins | Minimum Contents | Maximum Contents |
|---|---|---|
| TF | 0.1% | 0.3% |
| LTF | 0.0% | 0.2% |
| FOLR1 | 0.1% | 0.2% |
| CD14 | 0.1% | 0.2% |
| CIB1 | 0.1% | 0.4% |
| VPS25 | 0.0% | 0.4% |
| GNAS | 0.0% | 0.1%; |

the proteins in the mEV and their abundance ratios are shown in FIG. 8.

In some specific embodiments of the present invention, the mEV further comprises:

| | |
|---|---|
| APOA4 | 0.5% |
| CSN3 | 0.1% |
| LALBA | 0.3% |
| PEBP1 | 0.2% |
| SOD1 | 0.2% |
| LDHB | 0.2% |
| ANXA2 | 0.2% |
| MYO1C | 0.2%; |

the proteins in the mEV and their abundance ratios are shown in FIG. 9;
alternatively, the mEV further comprises:

| | |
|---|---|
| TF | 0.3% |
| LTF | 0.2% |
| FOLR1 | 0.2% |
| CD14 | 0.1% |
| CIB1 | 0.1% |
| VPS25 | 0.1% |
| GNAS | 0.1%; |

the proteins in the mEV and their abundance ratios are shown in FIG. 10.

In a third aspect, the present invention further provides a medicament comprising the mEV described above and a pharmaceutically acceptable excipient.

In a fourth aspect, the present invention further provides a pharmaceutical composition comprising the mEV described above and an additional active ingredient.

In a fifth aspect, the present invention further provides a method for using the mEV described above, comprising administering the mEV to a subject in need thereof.

In a sixth aspect, the present invention further provides a method for using the medicament described above, comprising administering the medicament to a subject in need thereof.

In a seventh aspect, the present invention further provides a method for using the pharmaceutical composition described above, comprising administering the pharmaceutical composition to a subject in need thereof.

In an eighth aspect, the present invention further provides a method for treating gastric ulcer, comprising administering to a subject in need thereof one or at least two of:
(I) the mEV,
(II) the medicament, or
(III) the pharmaceutical composition.

The applicants have found in research that milk-derived extracellular vesicles (mEVs) are rich in protein components facilitating reconstruction of gastric mucosa and immunoregulation, such as mucin, and most of the small nucleic acids (e.g., miRNAs) contained and loaded therein are closely associated with immunoregulation and anti-infection. Based on this, the applicants have developed the novel technology for preventing and treating gastric ulcer by using purified mEVs.

The beneficial effects of the present invention include, but are not limited to the following.
(1) The mEVs can be extracted from various dairy products such as fresh milk, raw milk, and cheese whey, which are high in contents, thereby allowing for unrestricted production in scale.
(2) Since the production process of mEVs does not require *in vitro* cell culture, the production cost is low.
(3) The mEVs can be administered orally.
(4) The mEVs have no safety risks, as demonstrated by the long-standing consumption of milk among a wide range of population.

### BRIEF DESCRIPTION OF DRAWINGS

FIG. 1 shows the concentration and particle size of the mEV particles in Example 1, as measured by NanoFCM.
FIG. 2 shows the morphology of the mEVs in Example 1, as observed by using a transmission electron microscope.
FIG. 3 shows the concentration and particle size of the mEV particles in Example 2, as measured by NanoFCM.
FIG. 4 shows the morphology of the mEVs in Example 2, as observed by a transmission electron microscope.
FIGs. 5 to 11 show the abundance ratios of proteins in the mEVs.

### DETAILED DESCRIPTION

The present invention discloses mEVs and use thereof in the manufacture of a medicament for preventing and/or treating gastric ulcer. Those skilled in the art can refer to the contents of this disclosure and appropriately improve the process parameters to realize the present invention. It should be particularly noted that, all similar substitutions and modifications are obvious to those skilled in the art, and they are deemed to be included in the present invention. The method and the use of the present invention have been described through the preferred examples, and it is obvious that the method and the use described herein may be changed or appropriately modified and combined to realize and apply the technology of the present invention by those skilled in the art without departing from the content, spirit and scope of the present invention.

The raw materials and reagents, used in the mEVs and use thereof in the manufacture of a medicament for preventing and/or treating gastric ulcer according to the present invention, are all commercially available. The present disclosure is further illustrated below in conjunction with examples.

### Interpretation of Terms:

**Gastric ulcer:** The gastric ulcer refers to a chronic ulcer located between the pylorus and cardia of the stomach. The peptic ulcer is a common, frequently occurred disease in the world. It is estimated that about 10% of people in the world have suffered from this disease in their lifetimes. Although the gastric ulcer can occur at any age, it is common in middle-aged and elderly people, with a male predominance in prevalence. The pathogenesis of gastric ulcer is the imbalance between the aggressive effect of gastric acid and pepsin and the defensive effect, due to the autodigestion of the mucosa caused by gastric acid in combination with the HP *(Helicobacter pylori)* infection, the self-protection of the gastric mucosal barrier is weakened and injured, which leads to erosion and then occurrence of ulcer.

**Extracellular vesicles/nanovesicles:** Extracellular vesicles are natural nanoscale vesicles (with a size of 30-150 nm) produced in cells and then released, which contain nucleic acids (genetic information) and proteins derived from their source cells. After binding to or being endocytosed by recipient cells, extracellular vesicles can release active molecules to activate downstream reactions and play a role in intercellular communication in the body. By engineering modification, extracellular vesicles being a natural intercellular communication tool, can be loaded with active molecules, and then delivered into recipient cells to exert activity. Especially in stem cell induction and differentiation, extracellular vesicles can be used to directly deliver transcription factors into stem cells, which avoids the risk of tumorigenesis associated with genomic changes caused by the conventional genetic engineering (such as viral infection) of cells for the high expression of transcription factors.

**Milk-derived extracellular vesicles (mEVs):** Milk-derived extracellular vesicles (mEVs) are biological nanoparticles formed by the bilayer phospholipid membrane, which can be extracted from milk in a large scale. Their natural functions include the delivery of bioactive molecules (such as proteins, and small nucleic acids) from the parent (female cow) to the gastrointestinal tract of the offspring (calf). Studies have confirmed that mEVs are resistant to digestive enzymes, low pH and other environmental factors in the digestive tract. Given the widespread consumption of milk, the long-term exposure of most people to mEVs (through milk consumption) suggests that mEVs are safe and tolerated in humans upon oral administration.

The present invention is further illustrated below in conjunction with examples.

### Example 1: Preparation of mEVs from Fresh Skimmed Milk by Ammonium Sulfate Precipitation Method and Verification of Therapeutic Effect of mEVs in Rat Gastric Ulcer Model

The objective was to prepare mEVs from fresh skimmed milk by ammonium sulfate precipitation method and verify their therapeutic effect in rat gastric ulcer model.

### Methods:

### Preparation of mEVs:

A 3M ammonium sulfate solution was prepared, and an equal volume of milk was slowly added to the ammonium sulfate solution with stirring, resulting in a final concentration of ammonium sulfate of 1.5 M. The mixture was left at room temperature for 3 h. After precipitates were generated, the mixture was filtered through a 400-mesh filter cloth, then filtering through a 0.8/0.45 µm capsule filter. Then, the resulting supernatant was concentrated using a 300 kD tangential flow filtration membrane cassette, and diafiltered for exchanging buffer into 1×PBS. The concentrated sample was refined through Capto Core700 (Cytiva) to remove free impurity proteins. Finally, the sample was concentrated to a target volume or concentration using a 750 kD hollow fiber tangential flow filtration column. The prepared mEV particles (A1 mEVs) were then analyzed by NanoFCM (NanoFCM, Xiamen) for measurement of concentration and particle size, transmission electron microscopy for morphological detection, and proteomic technology (WayenBio, Shanghai) for protein distribution and quantification.

### Animal Model:

SD rats were administered via gavage and randomly divided according to body weight into 6 groups: a blank control group (Group A, healthy rats), a model control group (Group B, the gastric ulcer model was established in rats and then the rats was administered with physiological saline via gavage), a positive drug control group (Group C, the gastric ulcer model was established in rats, and then the rats was administered with rabeprazole sodium as positive control drug via gavage at 20 mg/kg), a low-dose milk exosome group (Group D, rats were administered with mEVs via gavage at 10¹¹p/kg), an medium-dose milk exosome group (Group E, rats were administered with mEVs via gavage at 10¹²p/kg), and a high-dose milk exosome group (Group F, rats were administered with mEVs via gavage at 10¹³p/kg).

Administration frequency: qd×5 (continuous administration for 5 days after modeling).

Modeling: The rats were fasted for 16 h before modeling, with free access to water. All the rats, except those in the blank control group, were fixed on an iron wire mesh plate in a supine position and immersed in water within a constant temperature box at 20±1°C, with the water level maintained at the xiphoid process of the sternum.

Evaluation: After the dosing regimen was completed, rats were sacrificed through CO₂ inhalation. The abdomen was opened, the pylorus and cardia were ligated, and 10 mL of 2% formalin was infused into the stomach for 1 h of fixation. The gastric wall was opened by cutting along the greater curvature and flatly spread. The ulcerous lesions were counted under a dissecting microscope. The ulcer index was calculated, and the ulcer inhibition rate (%) was determined as: ulcer inhibition rate (%) = (average UI of the model control group - average UI of the administration group) / average UI of the model control group × 100%.

### Results:

### 1. Characterization

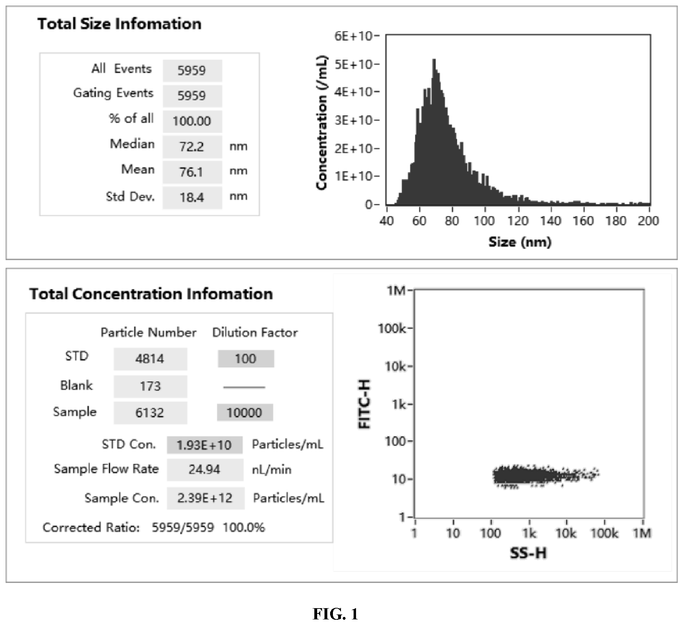

The concentration and particle size of mEV particles, as measured by NanoFCM, are shown in FIG. 1.

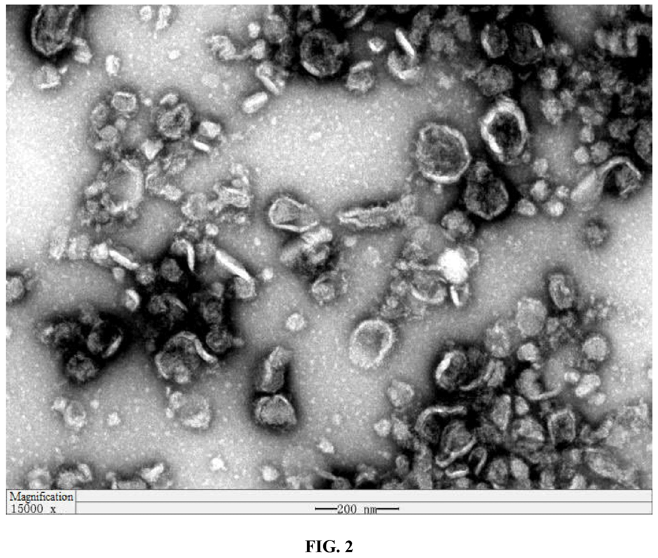

The morphology of mEVs, as detected by using a transmission electron microscope, is shown in FIG. 2.

### 2. Proteomic Analysis

The mEVs contained a large number of glycoproteins. Among them, glycosylation-dependent cell adhesion molecule 1 was the most abundant, which plays a role in adhesion and signaling between cells in tissues. The EV marker protein CD9 was also relatively abundant. In addition, the mEV also contained abundant apolipoprotein and immune-associated proteins.

**Table 1 Accession number, gene symbol and function description of top 50 proteins ranked by abundance in A1 mEVs**

| Accession | Gene Symbol | Description | Abundance (%) |
|---|---|---|---|
| P80195 | GLYCA M1 | Glycosylation-dependent cell adhesion molecule 1 OS=Bos taurus OX=9913 GN=GLYCAM1 PE=1 SV=2 | 10.6% |
| P18892 | BTN1A1 | Butyrophilin subfamily 1 member A1 OS=Bos taurus OX=9913 GN=BTN1A1 PE=1 SV=2 | 9.4% |
| P02754 | LGB | Beta-lactoglobulin OS=Bos taurus OX=9913 GN=LGB PE=1 SV=3 | 7.2% |
| Q95114 | MFGE8 | Lactadherin OS=Bos taurus OX=9913 GN=MFGE8 PE=1 SV=2 | 6.6% |
| P26201 | CD36 | Platelet glycoprotein 4 OS=Bos taurus OX=9913 GN=CD36 PE=1 SV=5 | 6.3% |
| P80457 | XDH | Xanthine dehydrogenase/oxidase OS=Bos taurus OX=9913 GN=XDH PE=1 SV=4 | 6.0% |
| Q4GZT4 | ABCG2 | ATP-binding cassette sub-family G member 2 OS=Bos taurus OX=9913 GN=ABCG2 PE=3 SV=2 | 4.5% |
| P10790 | FABP3 | Fatty acid-binding protein, heart OS=Bos taurus OX=9913 GN=FABP3 PE=1 SV=2 | 3.9% |
| Q27960 | SLC34A2 | Sodium-dependent phosphate transport protein 2B OS=Bos taurus OX=9913 GN=SLC34A2 PE=1 SV=1 | 3.8% |
| Q8MI01 | MUC15 | Mucin-15 OS=Bos taurus OX=9913 GN=MUC15 PE=1 SV=1 | 3.6% |
| Q71SP7 | FASN | Fatty acid synthase OS=Bos taurus OX=9913 GN=FASN PE=2 SV=1 | 3.1% |
| Q9XSG3 | IDH1 | Isocitrate dehydrogenase [NADP] cytoplasmic OS=Bos taurus OX=9913 GN=IDH1 PE=2 SV=1 | 2.3% |
| P30932 | CD9 | CD9 antigen OS=Bos taurus OX=9913 GN=CD9 PE=2 SV=2 | 2.2% |
| P15396 | ENPP3 | Ectonucleotide pyrophosphatase/phosphodiesterase family member 3 OS=Bos taurus OX=9913 GN=ENPP3 PE=1 SV=2 | 1.4% |
| Q8WML 4 | MUC1 | Mucin-1 OS=Bos taurus OX=9913 GN=MUC1 PE=1 SV=1 | 1.1% |
| P15497 | APOA1 | Apolipoprotein A-I OS=Bos taurus OX=9913 GN=APOA1 PE=1 SV=3 | 1.0% |
| P81287 | ANXA5 | Annexin A5 OS=Bos taurus OX=9913 GN=ANXA5 PE=1 SV=3 | 0.9% |
| Q0IIG8 | RAB18 | Ras-related protein Rab-18 OS=Bos taurus OX=9913 GN=RAB18 PE=2 SV=1 | 0.9% |
| P00974 | BPTI | Pancreatic trypsin inhibitor OS=Bos taurus OX=9913 PE=1 SV=2 | 0.8% |
| P81265 | PIGR | Polymeric immunoglobulin receptor OS=Bos taurus OX=9913 GN=PIGR PE=2 SV=1 | 0.7% |
| P62935 | PPIA | Peptidyl-prolyl cis-trans isomerase A OS=Bos taurus OX=9913 GN=PPIA PE=1 SV=2 | 0.7% |
| Q9TUM6 | PLIN2 | Perilipin-2 OS=Bos taurus OX=9913 GN=PLIN2 PE=2 SV=1 | 0.6% |
| P50397 | GDI2 | Rab GDP dissociation inhibitor beta OS=Bos taurus OX=9913 GN=GDI2 PE=2 SV=3 | 0.5% |
| Q05927 | NT5E | 5'-nucleotidase OS=Bos taurus OX=9913 GN=NT5E PE=1 SV=2 | 0.5% |
| Q32PJ2 | APOA4 | Apolipoprotein A-IV OS=Bos taurus OX=9913 GN=APOA4 PE=2 SV=1 | 0.5% |
| P02769 | ALB | Serum albumin OS=Bos taurus OX=9913 GN=ALB PE=1 SV=4 | 0.4% |
| P31976 | EZR | Ezrin OS=Bos taurus OX=9913 GN=EZR PE=1 SV=2 | 0.4% |
| P19120 | HSPA8 | Heat shock cognate 71 kDa protein OS=Bos taurus OX=9913 GN=HSPA8 PE=1 SV=2 | 0.4% |
| P62998 | RAC1 | Ras-related C3 botulinum toxin substrate 1 OS=Bos taurus OX=9913 GN=RAC1 PE=1 SV=1 | 0.4% |
| P07514 | CYB5R3 | NADH-cytochrome b5 reductase 3 OS=Bos taurus OX=9913 GN=CYB5R3 PE=1 SV=3 | 0.4% |
| Q3T0F5 | RAB7A | Ras-related protein Rab-7a OS=Bos taurus OX=9913 GN=RAB7A PE=2 SV=1 | 0.4% |
| P02668 | CSN3 | Kappa-casein OS=Bos taurus OX=9913 GN=CSN3 PE=1 SV=1 | 0.4% |
| Q2HJH2 | RAB1B | Ras-related protein Rab-1B OS=Bos taurus OX=9913 GN=RAB1B PE=2 SV=1 | 0.3% |
| Q95LA9 | TLR2 | Toll-like receptor 2 OS=Bos taurus OX=9913 GN=TLR2 PE=2 SV=1 | 0.3% |
| Q2KJ93 | CDC42 | Cell division control protein 42 homolog OS=Bos taurus OX=9913 GN=CDC42 PE=1 SV=1 | 0.3% |
| Q9XSK2 | CD63 | CD63 antigen OS=Bos taurus OX=9913 GN=CD63 PE=2 SV=4 | 0.3% |
| Q6VE48 | CD46 | Membrane cofactor protein OS=Bos taurus OX=9913 GN=CD46 PE=1 SV=2 | 0.3% |
| P00711 | LALBA | Alpha-lactalbumin OS=Bos taurus OX=9913 GN=LALBA PE=1 SV=2 | 0.3% |
| P19803 | ARHGDI A | Rho GDP-dissociation inhibitor 1 OS=Bos taurus OX=9913 GN=ARHGDIA PE=1 SV=3 | 0.3% |
| P68103 | EEF1A1 | Elongation factor 1-alpha 1 OS=Bos taurus OX=9913 GN=EEF1A1 PE=1 SV=1 | 0.3% |
| Q03247 | APOE | Apolipoprotein E OS=Bos taurus OX=9913 GN=APOE PE=1 SV=1 | 0.3% |
| P13696 | PEBP1 | Phosphatidylethanolamine-binding protein 1 OS=Bos taurus OX=9913 GN=PEBP1 PE=1 SV=2 | 0.2% |
| P61585 | RHOA | Transforming protein RhoA OS=Bos taurus OX=9913 GN=RHOA PE=1 SV=1 | 0.2% |
| P00442 | SOD1 | Superoxide dismutase [Cu-Zn] OS=Bos taurus OX=9913 GN=SOD1 PE=1 SV=2 | 0.2% |
| Q5EA88 | GPD1 | Glycerol-3-phosphate dehydrogenase [NAD(+)], cytoplasmic OS=Bos taurus OX=9913 GN=GPD1 PE=2 SV=3 | 0.2% |
| QSE9B1 | LDHB | L-lactate dehydrogenase B chain OS=Bos taurus OX=9913 GN=LDHB PE=2 SV=4 | 0.2% |
| Q3SYU2 | EEF2 | Elongation factor 2 OS=Bos taurus OX=9913 GN=EEF2 PE=2 SV=3 | 0.2% |
| Q2KJ44 | PTPA | Serine/threonine-protein phosphatase 2A activator OS=Bos taurus OX=9913 GN=PTPA PE=2 SV=1 | 0.2% |
| P04272 | ANXA2 | Annexin A2 OS=Bos taurus OX=9913 GN=ANXA2 PE=1 SV=2 | 0.2% |
| Q27966 | MYO1C | Unconventional myosin-Ic OS=Bos taurus OX=9913 GN=MYO1C PE=1 SV=3 | 0.2% |

The abundance ratios of proteins in mEVs are shown in FIG. 6.

### 3. Inhibition effect on gastric ulcer development

Compared with the model control group, mEVs exhibited a dose-dependent inhibitory effect on gastric ulcer development at various doses.

**Table 2 Gastric mucosal injury index of various administration groups**

| Groups | Number of Animals | Gastric Mucosal Injury Indexes (UI) |
|---|---|---|
| Saline Control | 6 | 0 |
| Model Control | 6 | 76.3±22.9 |
| Rabeprazole Sodium (20mg/kg) | 6 | 32.8±6.76* |
| A1 mEV (10¹¹particles/kg) | 6 | 66.0±19.5 |
| A1 mEV (10¹²particles/kg) | 6 | 58.2±22.5 |
| A1 mEV (10¹³particles/kg) | 6 | 41.5±6.32* |

| | | |
|---|---|---|
| The symbol * indicates a significant difference compared with the model control group (P<0.05). | | |

**Table 3 Ulcer inhibition rate (%) of various administration groups calculated according to Table 2 (Ulcer inhibition rate (%) = (UI of administration group - UI of model control group) / UI of model control group)**

| Groups | Ulcer Inhibition Rates (%) |
|---|---|
| Rabeprazole Sodium (20mg/kg) | 57 |
| A1 mEV (10¹¹particles/kg) | 13.5 |
| A1 mEV (10¹²particles/kg) | 23.7 |
| A1 mEV (10¹³particles/kg) | 45.6 |

### Example 2: Preparation of mEVs from Cheese Whey and Verification of Preventing Effect of mEVs on Gastric ulcer in Rat Gastric Ulcer Model

The objective was to prepare mEVs from cheese whey and verify their prophylactic effect on gastric ulcer in rat gastric ulcer model.

### Methods:

### Preparation of mEVs

Cheese whey was centrifuged at 5500 rpm for 10 min and a floating fat layer was removed. The remainder was filtered through a 400-mesh filter cloth, then filtering through a 0.8/0.45 µm capsule filter. Then, the resulting supernatant was concentrated using a 300 kD tangential flow filtration membrane cassette, and diafiltered for buffer exchange into 1×PBS. The concentrated sample was subjected to refined purification through Capto Core700 (Cytiva) to remove free impurity proteins. Finally, the sample was concentrated to a target volume or concentration using a 750 kD hollow fiber tangential flow filtration column. The prepared mEV particles were then analyzed by NanoFCM (NanoFCM, Xiamen) for measurement of concentration and particle size, transmission electron microscopy for morphological detection, and proteomic technology (WayenBio, Shanghai) for protein distribution and quantification.

### Animal Model

Preventive administration: The experiment was conducted as described in Example 1, except that the administration was performed prior to and during modeling.

### Results:

### 1. Characterization:

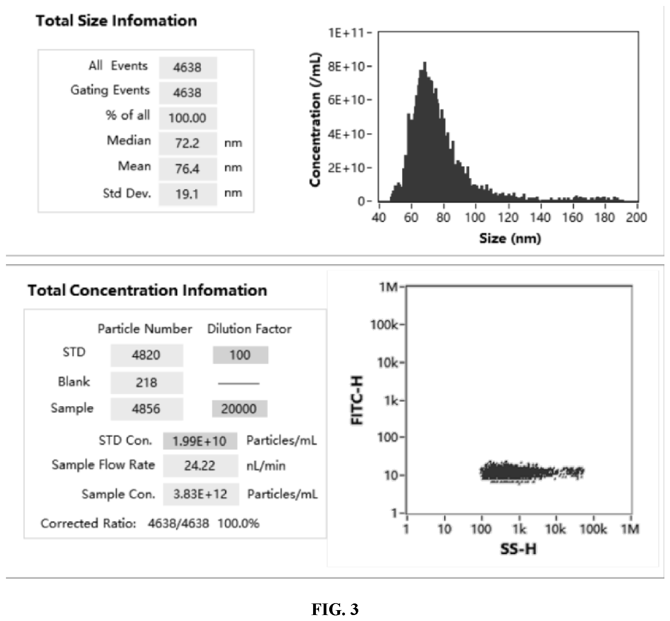

The concentration and particle size of mEVs, as measured by NanoFCM, are shown in FIG. 3.

The morphology of mEVs, as detected by using a transmission electron microscope, is shown in FIG. 4.

### 2. Proteomic analysis

mEVs contained a large number of glycoproteins. Among them, lactadherin was the most abundant, which has an anti-diarrheal effect. The EV marker protein CD9, CD81 and CD63, as well as some immune-associated proteins, apolipoproteins, and cytokines were also relatively abundant.

**Table 4 Accession number, gene symbol and function description of top 50 proteins ranked by abundance in mEVs**

| Accession | Gene Symbol | Description | Abundance (%) |
|---|---|---|---|
| Q95114 | MFGE8 | Lactadherin OS=Bos taurus OX=9913 GN=MFGE8 PE=1 SV=2 | 21.6% |
| P18892 | BTN1A1 | Butyrophilin subfamily 1 member A1 OS=Bos taurus OX=9913 GN=BTN1A1 PE=1 SV=2 | 14.8% |
| P80195 | GLYCAM1 | Glycosylation-dependent cell adhesion molecule 1 OS=Bos taurus OX=9913 GN=GLYCAM1 PE=1 SV=2 | 11.8% |
| P80457 | XDH | Xanthine dehydrogenase/oxidase OS=Bos taurus OX=9913 GN=XDH PE=1 SV=4 | 8.5% |
| P26201 | CD36 | Platelet glycoprotein 4 OS=Bos taurus OX=9913 GN=CD36 PE=1 SV=5 | 4.2% |
| Q4GZT4 | ABCG2 | ATP-binding cassette sub-family G member 2 OS=Bos taurus OX=9913 GN=ABCG2 PE=3 SV=2 | 3.1% |
| Q9TUM6 | PLIN2 | Perilipin-2 OS=Bos taurus OX=9913 GN=PLIN2 PE=2 SV=1 | 3.1% |
| P10790 | FABP3 | Fatty acid-binding protein, heart OS=Bos taurus OX=9913 GN=FABP3 PE=1 SV=2 | 2.8% |
| Q71 SP7 | FASN | Fatty acid synthase OS=Bos taurus OX=9913 GN=FASN PE=2 SV=1 | 2.7% |
| Q27960 | SLC34A2 | Sodium-dependent phosphate transport protein 2B OS=Bos taurus OX=9913 GN=SLC34A2 PE=1 SV=1 | 2.4% |
| P02754 | LGB | Beta-lactoglobulin OS=Bos taurus OX=9913 GN=LGB PE=1 SV=3 | 1.8% |
| Q9XSG3 | IDH1 | Isocitrate dehydrogenase [NADP] cytoplasmic OS=Bos taurus OX=9913 GN=IDH1 PE=2 SV=1 | 1.7% |
| Q8MI01 | MUC15 | Mucin-15 OS=Bos taurus OX=9913 GN=MUC15 PE=1 SV=1 | 1.4% |
| P30932 | CD9 | CD9 antigen OS=Bos taurus OX=9913 GN=CD9 PE=2 SV=2 | 1.2% |
| Q8WML4 | MUC1 | Mucin-1 OS=Bos taurus OX=9913 GN=MUC1 PE=1 SV=1 | 1.1% |
| P81265 | PIGR | Polymeric immunoglobulin receptor OS=Bos taurus OX=9913 GN=PIGR PE=2 SV=1 | 0.8% |
| P15396 | ENPP3 | Ectonucleotide pyrophosphatase/phosphodiesterase family member 3 OS=Bos taurus OX=9913 GN=ENPP3 PE=1 SV=2 | 0.8% |
| Q0IIG8 | RAB18 | Ras-related protein Rab-18 OS=Bos taurus OX=9913 GN=RAB18 PE=2 SV=1 | 0.6% |
| P00974 | BPTI | Pancreatic trypsin inhibitor OS=Bos taurus OX=9913 PE=1 SV=2 | 0.5% |
| P62935 | PPIA | Peptidyl-prolyl cis-trans isomerase A OS=Bos taurus OX=9913 GN=PPIA PE=1 SV=2 | 0.4% |
| P81287 | ANXA5 | Annexin A5 OS=Bos taurus OX=9913 GN=ANXA5 PE=1 SV=3 | 0.3% |
| Q05927 | NT5E | 5'-nucleotidase OS=Bos taurus OX=9913 GN=NT5E PE=1 SV=2 | 0.3% |
| Q29443 | TF | Serotransferrin OS=Bos taurus OX=9913 GN=TF PE=2 SV=1 | 0.3% |
| P15497 | APOA1 | Apolipoprotein A-I OS=Bos taurus OX=9913 GN=APOA1 PE=1 SV=3 | 0.3% |
| P19120 | HSPA8 | Heat shock cognate 71 kDa protein OS=Bos taurus OX=9913 GN=HSPA8 PE=1 SV=2 | 0.3% |
| P68103 | EEF1A1 | Elongation factor 1-alpha 1 OS=Bos taurus OX=9913 GN=EEF1A1 PE=1 SV=1 | 0.3% |
| Q95LA9 | TLR2 | Toll-like receptor 2 OS=Bos taurus OX=9913 GN=TLR2 PE=2 SV=1 | 0.3% |
| P07514 | CYB5R3 | NADH-cytochrome b5 reductase 3 OS=Bos taurus OX=9913 GN=CYB5R3 PE=1 SV=3 | 0.3% |
| Q32PJ2 | APOA4 | Apolipoprotein A-IV OS=Bos taurus OX=9913 GN=APOA4 PE=2 SV=1 | 0.3% |
| P31976 | EZR | Ezrin OS=Bos taurus OX=9913 GN=EZR PE=1 | 0.3% |
| P50397 | GDI2 | Rab GDP dissociation inhibitor beta OS=Bos taurus OX=9913 GN=GDI2 PE=2 SV=3 | 0.2% |
| Q3ZCD0 | CD81 | CD81 antigen OS=Bos taurus OX=9913 GN=CD81 PE=2 SV=1 | 0.2% |
| Q9XSK2 | CD63 | CD63 antigen OS=Bos taurus OX=9913 GN=CD63 PE=2 SV=4 | 0.2% |
| Q3T0F5 | RAB7A | Ras-related protein Rab-7a OS=Bos taurus OX=9913 GN=RAB7A PE=2 SV=1 | 0.2% |
| P24627 | LTF | Lactotransferrin OS=Bos taurus OX=9913 GN=LTF PE=1 SV=2 | 0.2% |
| P02702 | FOLR1 | Folate receptor alpha OS=Bos taurus OX=9913 GN=FOLR1 PE=1 SV=3 | 0.2% |
| P62998 | RAC1 | Ras-related C3 botulinum toxin substrate 1 OS=Bos taurus OX=9913 GN=RAC1 PE=1 SV=1 | 0.2% |
| Q2HJH2 | RAB1B | Ras-related protein Rab-1B OS=Bos taurus OX=9913 GN=RAB1B PE=2 SV=1 | 0.2% |
| Q3SYU2 | EEF2 | Elongation factor 2 OS=Bos taurus OX=9913 GN=EEF2 PE=2 SV=3 | 0.2% |
| Q6VE48 | CD46 | Membrane cofactor protein OS=Bos taurus OX=9913 GN=CD46 PE=1 SV=2 | 0.2% |
| Q2KJ93 | CDC42 | Cell division control protein 42 homolog OS=Bos taurus OX=9913 GN=CDC42 PE=1 SV=1 | 0.1% |
| Q95122 | CD14 | Monocyte differentiation antigen CD14 OS=Bos taurus OX=9913 GN=CD14 PE=2 SV=2 | 0.1% |
| Q17QE5 | CIB1 | Calcium and integrin-binding protein 1 OS=Bos taurus OX=9913 GN=CIB1 PE=2 SV=1 | 0.1% |
| Q5E9A6 | VPS25 | Vacuolar protein-sorting-associated protein 25 OS=Bos taurus OX=9913 GN=VPS25 PE=2 SV=1 | 0.1% |
| P19803 | ARHGDIA | Rho GDP-dissociation inhibitor 1 OS=Bos taurus OX=9913 GN=ARHGDIA PE=1 SV=3 | 0.1% |
| Q5EA88 | GPD1 | Glycerol-3-phosphate dehydrogenase [NAD(+)], cytoplasmic OS=Bos taurus OX=9913 GN=GPD1 PE=2 SV=3 | 0.1% |
| P61585 | RHOA | Transforming protein RhoA OS=Bos taurus OX=9913 GN=RHOA PE=1 SV=1 | 0.1% |
| Q2KJ44 | PTPA | Serine/threonine-protein phosphatase 2A activator OS=Bos taurus OX=9913 GN=PTPA PE=2 SV=1 | 0.1% |
| Q03247 | APOE | Apolipoprotein E OS=Bos taurus OX=9913 GN=APOE PE=1 SV=1 | 0.1% |
| P04896 | GNAS | Guanine nucleotide-binding protein G(s) subunit alpha isoforms short OS=Bos taurus OX=9913 GN=GNAS PE=1 SV=1 | 0.1% |

The proteins in mEVs and their abundance ratios are shown in FIG. 11.

### 3. Inhibition Effect on Gastric Ulcer Development

The mEVs exhibited different degrees of inhibitory effect on gastric ulcer development at various doses. Both the high-dose administration group (10¹³particles/kg) and the positive drug rabeprazole sodium (20 mg/kg) exhibited significant inhibition effects on ulcer development.

**Table 5 Gastric mucosal injury index of various administration groups**

| Groups | Number of Animals | Gastric Mucosal Injury Indexes (UI) |
|---|---|---|
| Saline Control | 6 | 0 |
| Model Control | 6 | 76.0±16.4 |
| Rabeprazole Sodium (20mg/kg) | 6 | 31.8±18.7* |
| B3 mEV (10¹¹particles/kg) | 6 | 57.3±21.2 |
| B3 mEV (10¹²particles/kg) | 6 | 51.6±19.0 |
| B3 mEV (10¹³particles/kg) | 6 | 49.8±13.6* |

| | | |
|---|---|---|
| The symbol * indicates a significant difference compared with the model control group (P<0.05). | | |

**Table 6 Ulcer inhibition rate (%) of various administration groups calculated according to Table 5 (Ulcer inhibition rate (%) = (UI of the administration group - UI of the model control group) / UI of the model control group)**

| Groups | Ulcer Inhibition Rates (%) |
|---|---|
| Rabeprazole Sodium (20mg/kg) | 58.2 |
| B3 mEV (10¹¹particles/kg) | 24.6 |
| B3 mEV (10¹²particles/kg) | 32.1 |
| B3 mEV (10¹³particles/kg) | 34.5 |

The mEV and use thereof in the manufacture of a medicament for preventing and/or treating gastric ulcer provided by the present invention are described in detail above. The principle and implementation of the present invention are illustrated by using specific embodiments herein. The above descriptions of the examples are only used to facilitate understanding of the method and the core idea of the present invention. It should be noted that, several improvements and modifications may be made to the present invention by those skilled in the art without departing from the principle of the present invention, and the improvements and modifications also fall within the protection scope of the claims.

## Claims

1. Use of a milk-derived extracellular vesicle (mEV) in the manufacture of a medicament for preventing and/or treating gastric ulcer.

2. The use according to claim 1, wherein the method of preparing the mEV comprises:
step 1: pre-treating a raw material, filtering the raw material through a 400-mesh filter cloth, then filtering through a 0.8/0.45 µm capsule filter, and collecting a filtrate,
step 2: concentrating the filtrate with a 300 kD tangential flow filtration membrane cassette, and diafiltering to exchange buffer into 1×PBS,
step 3: purifying the concentrated sample through Capto Core 700(Cytiva) for further purification and removal of a free impurity protein, and
step 4: concentrating with a 750 kD hollow fiber tangential flow filtration column into a target volume or concentration.

3. The use according to claim 2, wherein the raw material includes fresh skimmed milk or cheese whey;
when the raw material is fresh skimmed milk, the pre-treating comprises preparing a 3 M ammonium sulfate solution, slowly adding an equal volume of the fresh skimmed milk to the ammonium sulfate solution with stirring to achieve a final concentration of ammonium sulfate of 1.5 M, and standing at room temperature for 3 h to allow precipitation;
when the raw material is cheese whey, the pre-treating comprises centrifuging the cheese whey at 5500 rpm for 10 min and removing a floating fat layer.

4. The use according to any one of claims 1 to 3, wherein the mEV comprises one or at least two of glycoprotein, extracellular vesicle (EV) marker protein, apolipoprotein, immune-associated protein and cytokine.

5. The use according to claim 4, wherein the mEV comprises one or at least two of:
GLYCAM1, BTN1A1, LGB, MFGE8, CD36, XDH, ABCG2, FABP3, SLC34A2, MUC15, FASN, IDH1, CD9, ENPP3, MUC1, APOA1, ANXA5, RAB18, BPTI, PIGR, PPIA, PLIN2, GDI2, NT5E, ALB, EZR, HSPA8, RAC1, CYB5R3, RAB7A, RAB1B, TLR2, CDC42, CD63, CD46, ARHGDIA, EEF1A1, APOE, RHOA, GPD1, EEF2 or PTPA.

6. The use according to claim 5, wherein the mEV comprises:
| Proteins | Minimum Contents | Maximum Contents |
|---|---|---|
| GLYCAM1 | 5.3% | 36.2% |
| BTN1A1 | 9.4% | 19.3% |
| LGB | 1.8% | 8.4% |
| MFGE8 | 5.0% | 21.6% |
| CD36 | 3.3% | 7.9% |
| XDH | 3.7% | 8.8% |
| ABCG2 | 1.9% | 4.7% |
| FABP3 | 2.5% | 3.9% |
| SLC34A2 | 1.5% | 4.0% |
| MUC15 | 1.1% | 3.6% |
| FASN | 1.7% | 6.3% |
| IDH1 | 1.5% | 2.7% |
| CD9 | 0.6% | 2.4% |
| ENPP3 | 0.4% | 1.6% |
| MUC1 | 0.4% | 1.9% |
| APOA1 | 0.1% | 3.3% |
| ANXA5 | 0.3% | 1.0% |
| RAB18 | 0.3% | 0.9% |
| BPTI | 0.5% | 2.3% |
| PIGR | 0.4% | 1.1% |
| PPIA | 0.2% | 0.7% |
| PLIN2 | 0.6% | 3.1% |
| GDI2 | 0.1% | 0.5% |
| NT5E | 0.3% | 0.5% |
| ALB | 0.0% | 0.7% |
| EZR | 0.1% | 1.1% |
| HSPA8 | 0.2% | 0.6% |
| RAC1 | 0.1% | 0.4% |
| CYB5R3 | 0.1% | 0.4% |
| RAB7A | 0.1% | 0.4% |
| RAB1B | 0.1% | 0.3% |
| TLR2 | 0.1% | 0.4% |
| CDC42 | 0.1% | 0.3% |
| CD63 | 0.0% | 0.7% |
| CD46 | 0.1% | 0.3% |
| ARHGDIA | 0.1% | 1.5% |
| EEF1A1 | 0.1% | 0.5% |
| APOE | 0.1% | 1.2% |
| RHOA | 0.1% | 0.2% |
| GPD1 | 0.1% | 0.2% |
| EEF2 | 0.1% | 0.3% |
| PTPA | 0.1% | 0.2% |

7. The use according to claim 5, wherein the mEV comprises:
| | |
|---|---|
| GLYCAM1 | 10.6% |
| BTN1A1 | 9.4% |
| LGB | 7.2% |
| MFGE8 | 6.6% |
| CD36 | 6.3% |
| XDH | 6.0% |
| ABCG2 | 4.5% |
| FABP3 | 3.9% |
| SLC34A2 | 3.8% |
| MUC15 | 3.6% |
| FASN | 3.1% |
| IDH1 | 2.3% |
| CD9 | 2.2% |
| ENPP3 | 1.4% |
| MUC1 | 1.1% |
| APOA1 | 1.0% |
| ANXA5 | 0.9% |
| RAB18 | 0.9% |
| BPTI | 0.8% |
| PIGR | 0.7% |
| PPIA | 0.7% |
| PLIN2 | 0.6% |
| GDI2 | 0.5% |
| NT5E | 0.5% |
| APOA4 | 0.5% |
| ALB | 0.4% |
| EZR | 0.4% |
| HSPA8 | 0.4% |
| RAC1 | 0.4% |
| CYB5R3 | 0.4% |
| RAB7A | 0.4% |
| CSN3 | 0.4% |
| RAB1B | 0.3% |
| TLR2 | 0.3% |
| CDC42 | 0.3% |
| CD63 | 0.3% |
| CD46 | 0.3% |
| LALBA | 0.3% |
| ARHGDIA | 0.3% |
| EEF1A1 | 0.3% |
| APOE | 0.3% |
| PEBP1 | 0.2% |
| RHOA | 0.2% |
| SOD1 | 0.2% |
| GPD1 | 0.2% |
| LDHB | 0.2% |
| EEF2 | 0.2% |
| PTPA | 0.2% . |

8. The use according to any one of claims 5 to 7, wherein the mEV further comprises:
(I) one or at least two of APOA4, CSN3, LALBA, PEBP1, SOD1, LDHB, ANXA2 or MYO1C; and/or
(II) one or at least two of TF, LTF, FOLR1, CD14, CIB1, VPS25 or GNAS.

9. The use according to claim 8, wherein the mEV further comprises:
| Proteins | Minimum Contents | Maximum Contents |
|---|---|---|
| APOA4 | 0.0% | 0.7% |
| CSN3 | 0.0% | 0.4% |
| LALBA | 0.1% | 0.4% |
| PEBP1 | 0.0% | 0.2% |
| SOD1 | 0.1% | 0.5% |
| LDHB | 0.1% | 0.3% |
| ANXA2 | 0.1% | 0.2% |
| MYO1C | 0.1% | 0.2% |
or further comprises:
| Proteins | Minimum Contents | Maximum Contents |
|---|---|---|
| TF | 0.1% | 0.3% |
| LTF | 0.0% | 0.2% |
| FOLR1 | 0.1% | 0.2% |
| CD14 | 0.1% | 0.2% |
| CIB1 | 0.1% | 0.4% |
| VPS25 | 0.0% | 0.4% |
| GNAS | 0.0% | 0.1% |

10. The use according to claim 8, wherein the mEV further comprises:
| | |
|---|---|
| APOA4 | 0.5% |
| CSN3 | 0.1% |
| LALBA | 0.3% |
| PEBP1 | 0.2% |
| SOD1 | 0.2% |
| LDHB | 0.2% |
| ANXA2 | 0.2% |
| MYO1C | 0.2% |
or further comprises:
| | |
|---|---|
| TF | 0.3% |
| LTF | 0.2% |
| FOLR1 | 0.2% |
| CD14 | 0.1% |
| CIB1 | 0.1% |
| VPS25 | 0.1% |
| GNAS | 0.1% |

11. An mEV, which is manufactured by a method comprising:
step 1: pre-treating a raw material, filtering the raw material through a 400-mesh filter cloth, then filtering through a 0.8/0.45 µm capsule filter, and collecting a filtrate,
step 2: concentrating the filtrate with a 300 kD tangential flow filtration membrane cassette, and diafiltering to exchange buffer into 1×PBS,
step 3: purifying the concentrated sample through Capto Core 700(Cytiva) for further purification and removal of a free impurity protein, and
step 4: concentrating with a 750 kD hollow fiber tangential flow filtration column into a target volume or concentration.

12. The mEV according to claim 11, wherein the raw material includes fresh skimmed milk or cheese whey;
when the raw material is fresh skimmed milk, the pre-treating comprises preparing a 3 M ammonium sulfate solution, slowly adding an equal volume of the fresh skimmed milk to the ammonium sulfate solution with stirring to achieve a final concentration of ammonium sulfate of 1.5 M, and standing at room temperature for 3 h to allow precipitation;
when the raw material is cheese whey, the pre-treating comprises centrifuging the cheese whey at 5500 rpm for 10 min and removing a floating fat layer.

13. The mEV according to claims 11 or 12, which comprises one or at least two of glycoprotein, EV marker protein, apolipoprotein, immune-associated protein and cytokine.

14. The mEV according to claim 13, which comprises one or at least two of:
GLYCAM1, BTN1A1, LGB, MFGE8, CD36, XDH, ABCG2, FABP3, SLC34A2, MUC15, FASN, IDH1, CD9, ENPP3, MUC1, APOA1, ANXA5, RAB18, BPTI, PIGR, PPIA, PLIN2, GDI2, NT5E, ALB, EZR, HSPA8, RAC1, CYB5R3, RAB7A, RAB1B, TLR2, CDC42, CD63, CD46, ARHGDIA, EEF1A1, APOE, RHOA, GPD1, EEF2or PTPA.

15. The mEV according to claim 13 or 14, which comprises:
| Proteins | Minimum Contents | Maximum Contents |
|---|---|---|
| GLYCAM1 | 5.3% | 36.2% |
| BTN1A1 | 9.4% | 19.3% |
| LGB | 1.8% | 8.4% |
| MFGE8 | 5.0% | 21.6% |
| CD36 | 3.3% | 7.9% |
| XDH | 3.7% | 8.8% |
| ABCG2 | 1.9% | 4.7% |
| FABP3 | 2.5% | 3.9% |
| SLC34A2 | 1.5% | 4.0% |
| MUC15 | 1.1% | 3.6% |
| FASN | 1.7% | 6.3% |
| IDH1 | 1.5% | 2.7% |
| CD9 | 0.6% | 2.4% |
| ENPP3 | 0.4% | 1.6% |
| MUC1 | 0.4% | 1.9% |
| APOA1 | 0.1% | 3.3% |
| ANXA5 | 0.3% | 1.0% |
| RAB18 | 0.3% | 0.9% |
| BPTI | 0.5% | 2.3% |
| PIGR | 0.4% | 1.1% |
| PPIA | 0.2% | 0.7% |
| PLIN2 | 0.6% | 3.1% |
| GDI2 | 0.1% | 0.5% |
| NT5E | 0.3% | 0.5% |
| ALB | 0.0% | 0.7% |
| EZR | 0.1% | 1.1% |
| HSPA8 | 0.2% | 0.6% |
| RAC1 | 0.1% | 0.4% |
| CYB5R3 | 0.1% | 0.4% |
| RAB7A | 0.1% | 0.4% |
| RAB1B | 0.1% | 0.3% |
| TLR2 | 0.1% | 0.4% |
| CDC42 | 0.1% | 0.3% |
| CD63 | 0.0% | 0.7% |
| CD46 | 0.1% | 0.3% |
| ARHGDIA | 0.1% | 1.5% |
| EEF1A1 | 0.1% | 0.5% |
| APOE | 0.1% | 1.2% |
| RHOA | 0.1% | 0.2% |
| GPD1 | 0.1% | 0.2% |
| EEF2 | 0.1% | 0.3% |
| PTPA | 0.1% | 0.2% |

16. The mEV according to claim 13 or 14, which comprises:
| | |
|---|---|
| GLYCAM1 | 10.6% |
| BTN1A1 | 9.4% |
| LGB | 7.2% |
| MFGE8 | 6.6% |
| CD36 | 6.3% |
| XDH | 6.0% |
| ABCG2 | 4.5% |
| FABP3 | 3.9% |
| SLC34A2 | 3.8% |
| MUC15 | 3.6% |
| FASN | 3.1% |
| IDH1 | 2.3% |
| CD9 | 2.2% |
| ENPP3 | 1.4% |
| MUC1 | 1.1% |
| APOA1 | 1.0% |
| ANXA5 | 0.9% |
| RAB18 | 0.9% |
| BPTI | 0.8% |
| PIGR | 0.7% |
| PPIA | 0.7% |
| PLIN2 | 0.6% |
| GDI2 | 0.5% |
| NT5E | 0.5% |
| APOA4 | 0.5% |
| ALB | 0.4% |
| EZR | 0.4% |
| HSPA8 | 0.4% |
| RAC1 | 0.4% |
| CYB5R3 | 0.4% |
| RAB7A | 0.4% |
| CSN3 | 0.4% |
| RAB1B | 0.3% |
| TLR2 | 0.3% |
| CDC42 | 0.3% |
| CD63 | 0.3% |
| CD46 | 0.3% |
| LALBA | 0.3% |
| ARHGDIA | 0.3% |
| EEF1A1 | 0.3% |
| APOE | 0.3% |
| PEBP1 | 0.2% |
| RHOA | 0.2% |
| SOD1 | 0.2% |
| GPD1 | 0.2% |
| LDHB | 0.2% |
| EEF2 | 0.2% |
| PTPA | 0.2% |

17. The mEV according to any one of claims 13 to 16, which further comprises:
(I) one or at least two of APOA4, CSN3, LALBA, PEBP1, SOD1, LDHB, ANXA2or MYO1C; and/or
(II) one or at least two of TF, LTF, FOLR1, CD14, CIB1, VPS25 or GNAS.

18. The mEV according to claim 17, which further comprises:
| Proteins | Minimum Contents | Maximum Contents |
|---|---|---|
| APOA4 | 0.0% | 0.7% |
| CSN3 | 0.0% | 0.4% |
| LALBA | 0.1% | 0.4% |
| PEBP1 | 0.0% | 0.2% |
| SOD1 | 0.1% | 0.5% |
| LDHB | 0.1% | 0.3% |
| ANXA2 | 0.1% | 0.2% |
| MYO1C | 0.1% | 0.2% |
or further comprises:
| Proteins | Minimum Contents | Maximum Contents |
|---|---|---|
| TF | 0.1% | 0.3% |
| LTF | 0.0% | 0.2% |
| FOLR1 | 0.1% | 0.2% |
| CD14 | 0.1% | 0.2% |
| CIB1 | 0.1% | 0.4% |
| VPS25 | 0.0% | 0.4% |
| GNAS | 0.0% | 0.1% |

19. The mEV according to claim 17, which further comprises:
| | |
|---|---|
| APOA4 | 0.5% |
| CSN3 | 0.1% |
| LALBA | 0.3% |
| PEBP1 | 0.2% |
| SOD1 | 0.2% |
| LDHB | 0.2% |
| ANXA2 | 0.2% |
| MYO1C | 0.2% |
or further comprises:
| | |
|---|---|
| TF | 0.3% |
| LTF | 0.2% |
| FOLR1 | 0.2% |
| CD14 | 0.1% |
| CIB1 | 0.1% |
| VPS25 | 0.1% |
| GNAS | 0.1% |

20. A medicament, comprising the mEV according to any one of claims 11 to 19 and a pharmaceutically acceptable excipient.

21. A pharmaceutical composition, comprising the mEV according to any one of claims 11 to 19 and an additional active ingredient.

22. A method for using the mEV according to any one of claims 11 to 19, comprising administering the mEV to a subject in need thereof.

23. A method for using the medicament according to claim 20, comprising administering the medicament to a subject in need thereof.

24. A method for using the pharmaceutical composition according to claim 21, comprising administering the pharmaceutical composition to a subject in need thereof.

25. A method for treating gastric ulcer, comprising administering to a subject in need thereof one or at least two of:
(I) the mEV according to any one of claims 11 to 19,
(II) the medicament according to claim 20, or
(III) the pharmaceutical composition according to claim 21.
